# EUROPEAN PATENT SPECIFICATION

(11) **EP 3 030 223 B1**
(45) Date of publication and mention of the grant of the patent: **21.11.2018**
(21) Application number: 14835053.1
(22) Date of filing: 07.08.2014
(51) Int. Cl.: A61K 8/92, A61K 8/891, A61Q 15/00, A61K 8/06, A61K 8/894

(54) **A SOFT SOLID ANTIPERSPIRANT DEODORANT COMPOSITION**
WEICHE UND FESTE SCHWEISSHEMMENDE ZUSAMMENSETZUNG
COMPOSITION DÉODORANTE ANTITRANSPIRANTE SOLIDE MOLLE

(30) Priority: 08.08.2013 US 201313962528
(43) Date of publication of application: 15.06.2016
(73) Proprietor: Henkel IP & Holding GmbH, 40589 Düsseldorf (DE)
(72) Inventor: GE, Haiyan, Valencia, California 91354 (US); TSOTSOS, Amy, Scottsdale, Arizona 85253 (US)
(74) Representative: Henkel IP Department
(86) International application number: PCT/US2014/050215
(87) International publication number: WO 2015/021321

(56) References cited:
- WO-A1-00/44339
- US-A1- 2002 001 572
- US-A1- 2005 238 598
- US-A1- 2008 241 089
- US-A1- 2011 212 144
- US-A1- 2013 189 208
- US-B2- 7 776 347
- DATABASE GNPD [Online] MINTEL; 31 August 2011 (2011-08-31), "Soft Deodorant Stick", XP002764499, Database accession no. 1602341
- "Paraffin", GNPD Mintel , 31 August 2011 (2011-08-31), XP002764500, Retrieved from the Internet: URL:http://www.gnpd.com/sinatra/ingredient s/19382/ [retrieved on 2016-11-22]

## Description

### CROSS-REFERENCE TO RELATED APPLICATIONS

The present application claims priority to U. S. Utility Application 13/962,528 filed August 8, 2013 and entitled "SOFT SOLID ANTIPERSPIRANT DEODORANT COMPOSITION", which is incorporated herein.

### FIELD OF THE INVENTION

The present disclosure generally relates to antiperspirant compositions and methods for preparing antiperspirant compositions, and more particularly relates to antiperspirant compositions with microcrystalline wax structurants and paraffin wax structurants and methods for preparing the same.

### BACKGROUND OF THE INVENTION

Antiperspirants are popular personal care products used to prevent or eliminate perspiration and body odor caused by perspiration. Antiperspirant products, including for example sticks, emulsions, aerosol sprays, and roll-on antiperspirants are desired by a large majority of the population because of the presence of active antiperspirant compounds that minimize or prevent the secretion of perspiration by blocking or plugging ducts of sweat-secreting glands, such as those located at the underarms. Antiperspirants typically include an active antiperspirant compound in a carrier that permits the antiperspirant product to be applied to the skin by swiping or rubbing the stick across the skin, typically of the underarm. Upon application of the antiperspirant product, the carrier coats the skin or evaporates, releasing the active antiperspirant compound from the antiperspirant product upon exposure to moisture to form plugs in the sweat ducts.

Active antiperspirant compounds reduce underarm wetness and odor by migrating into openings of the sweat gland ducts and reacting with proteins therein to form antiperspirant plugs, which mechanically prevent sweat from escaping the ducts. One type of sweat gland in the underarm region is the apocrine sweat gland, which terminates and secretes at the top of hair follicles. Thus, active antiperspirant compounds are configured to migrate into the hair follicle to access the apocrine sweat gland duct and block secretion. Another type of sweat gland is the eccrine sweat gland, which opens directly onto the skin. Eccrine sweat is responsible for the largest volume of sweat that causes underarm wetness. As with apocrine sweat glands, active antiperspirant compounds migrate into the eccrine sweat gland openings and form plugs, which reduce underarm wetness.

Some antiperspirant compositions used in the stick type include one or multiple structurant compounds, which are included to provide good "glide" qualities (i.e., ease of application when the stick is moved across the underarm region) and to minimize residue deposits on the skin. Such structurant compounds include intermediate molecular weight polyethylenes, such as polyethylenes having an average molecular weight between about 360 Daltons and 460 Daltons.

Other antiperspirant compositions are incorporated into soft solids. A soft solid is a composition that appears to be solid, but acts as a fluid when a force is applied to the composition. Such soft solids have sufficient rigidity that they are not observed by the human eye to flow, but they are deformable by hand pressure and can be extruded from a container through apertures formed in a container. For example, some antiperspirant compositions are supplied in a container that can be squeezed or otherwise forced to flow out. Such antiperspirant deodorant products on the market are anhydrous soft solids in which an active powder antiperspirant ingredient is dispersed in volatile and non-volatile fluids thickened by structurants. These products rub in quickly, are non-tacky, and deliver high concentration of the active ingredient to the targeted sites. However, such soft solids can leave white residue on skin in form of flakes. US 2001/0212144 discloses antiperspirant compositions in the form of solid water-in-oil emulsions comprising dimethicone copolyols as emulsifiers and a particular wax as a structurant.

Accordingly, it is desirable to provide an antiperspirant soft solid that exhibits good antiperspirant qualities without leaving white residue behind. Further, it is desirable to provide methods for preparing such antiperspirant soft solid. Still further, other desirable features and characteristics of the present disclosure will become apparent from the subsequent detailed description and the appended claims, taken in conjunction with the accompanying drawings and background.

### BRIEF SUMMARY OF THE INVENTION

An antiperspirant composition according to the invention includes a water-in-oil emulsion having structurants and an emulsifier where the structurants include microcrystalline wax and paraffin wax at a microcrystalline wax:paraffin wax ratio of 1:1 to 1:5, wherein the paraffin wax is 6.0 to 8.0 weight percent of the composition and the microcrystalline wax is 4.0 to 6.0 weight percent of the composition.

### BRIEF DESCRIPTION OF THE DRAWINGS

The present invention will hereinafter be described in conjunction with the following drawing figures, wherein like numerals denote like elements, and
FIG. 1 is a diagram of a container of an exemplary soft solid antiperspirant composition according to the principles described herein; and
FIG. 2 is a diagram of an exemplary method for making a soft solid antiperspirant composition according to the principles described herein.

### DETAILED DESCRIPTION OF THE INVENTION

The following detailed description of the invention is merely exemplary in nature and is not intended to limit the invention or the application and uses of the invention. Furthermore, there is no intention to be bound by any theory presented in the preceding background of the invention or the following detailed description of the invention.

The various examples contemplated herein relate to antiperspirant compositions and methods for preparing antiperspirant compositions. The various examples of the compositions exhibit good antiperspirant quality without leaving a white residue behind. The antiperspirant composition includes a water-in-oil emulsion that forms a soft solid that is composed of small droplets of a water phase that are dispersed throughout an oil phase. The structurants used herein prevent the soft solid from solidifying by forming a non-continuous crystalline structure. Such a state allows the soft solid to be applied to the appropriate locations in a soft solid state through an appropriate applicator.

### Water Phase

Antiperspirant compositions in accordance with the principles described herein include a water phase. The water phase includes water and one or multiple water-soluble compounds. In one example, the water phase includes water, an active antiperspirant compound, and a carrier.

The active antiperspirant compound contains at least one active ingredient. For example, the active antiperspirant compound may be metal salts, that, as noted above, may reduce perspiration by diffusing through the sweat ducts of apocrine glands and eccrine glands and hydrolyzing in the sweat ducts. As a result, such metal salts combine with proteins to form an amorphous metal hydroxide agglomerate, plugging the sweat ducts so perspiration cannot diffuse to the skin surface.

Some active antiperspirant compounds that may be used in the antiperspirant composition include astringent metallic salts. For example, such astringent metallic salts may include inorganic and organic salts of aluminum, zirconium, and zinc, including tetra- and octa- salts, as well as mixtures thereof. Other such compounds may include aluminum-containing and/or zirconium-containing salts or materials, such as aluminum halides, aluminum chlorohydrates, aluminum hydroxyhalides, zirconyl oxyhalides, zirconyl hydroxyhalides, and mixtures thereof. In one example, aluminum salts may include those having the general formula Al₂(OH)ₐCl_{b} x (H₂O), wherein a is from 2 to about 5; a and b total to about 6; x is from 1 to about 6; and wherein a, b, and x may have non-integer values. In one example, zirconium salts may include those having the general formula ZrO(OH)2-ₐClₐ x (H₂O), wherein a is from about 1.5 to about 1.87, x is from about 1 to about 7, and wherein a and x may both have non-integer values. The zirconium salts may have complexes that additionally contain aluminum and glycine, commonly referred to as ZAG complexes. These ZAG complexes contain aluminum chlorohydroxide and zirconyl hydroxy chloride conforming to the above-described formulas.

Examples of active antiperspirant compounds suitable for use in the various products contemplated herein include aluminum dichlorohydrate, aluminum-zirconium octachlorohydrate, aluminum sesquichlorohydrate, aluminum chlorohydrex propylene glycol complex, aluminum dichlorohydrex propylene glycol complex, aluminum sesquichlorohydrex propylene glycol complex, aluminum chlorohydrex polyethylene glycol complex, aluminum dichlorohydrex polyethylene glycol complex, aluminum sesquichlorohydrex polyethylene glycol complex, aluminum-zirconium trichlorohydrate, aluminum zirconium tetrachlorohydrate, aluminum zirconium pentachlorohydrate, aluminum zirconium octachlorohydrate, aluminum zirconium trichlorohydrex glycine complex, aluminum zirconium tetrachlorohydrex glycine complex, aluminum zirconium pentachlorohydrex glycine complex, aluminum zirconium octachlorohydrex glycine complex, zirconium chlorohydrate, aluminum chloride, aluminum sulfate buffered, and the like, and mixtures thereof. In one example, the active antiperspirant compound is aluminum zirconium pentachlorohydrex glycine complex or aluminum zirconium trichlorohydrex glycine complex.

Any appropriate carrier may be used in accordance with the principles described herein. For example, the carrier may be propylene glycol, which may be purchased from Dow Corning, which is Midland, Michigan, U.S.A. In some example, water may be used as a carrier or a portion of the carrier.

Table 1 below illustrates a first example and a second example of a water phase of a soft solid antiperspirant deodorant. Each of the ingredients and compound amounts is indicated in weight percents of the overall soft solid antiperspirant composition.

**(Table 1)**

| Function | Ingredients and Compounds | 1^{st} Example Wt% | 2^{nd} Example Wt% |
|---|---|---|---|
| Active | AAZG-3109 - Aluminum Zirconium Octachlorohydrex GLY | 67.1141 | |
| Active | Ultra Zag 88L-Aluminum Zirconium Tetrachlorohydrex GLY | | 62.5 |
| Carrier | Propylene Glycol | 3.9859 | 5.6 |
| Carrier | Water | | 3 |
| Sum: | | 71.1 | 71.1 |

The active compositions in Table 1 may be purchased from Summit Reheis, which is located in Huguenot, New York, U.S.A. In the examples illustrated in Table 1, the active compositions constitute over 67.0 weight percent of the overall soft solid antiperspirant composition while the carrier is just under 4.0 weight percent of the overall soft solid antiperspirant composition. However, while the active composition is over 67.0 weight percent of the overall composition, just a portion of the active composition constitutes an active ingredient. For example, just 29.8 weight percent of AAZG 3109 is an active ingredient, which causes the active ingredient to be 20.0 weight percent in the overall composition. Further, the active ingredient in Ultra Zag 88L is 32.0 weight percent of this active compound, making the overall active ingredient to also be 20.0 weight percent of the overall composition.

To prepare the water phase, the components thereof are combined and heated. For example, the components can be combined and heated to a temperature of about 60°C, 65°C, 70°C, 75°C, 80°C, 85°C or any temperature thereinbetween. In some examples, the water phase is heated to between 64.0 °C and 66.0 °C before mixing the water phase with the oil phase. Further, the components of the water phase can be mechanically agitated to promote solubilization, such as by moderate stirring. As such, the water phase, when its constituents are combined, may total about 60.0 weight percent to about 75.0 weight percent of the overall antiperspirant composition.

### Oil Phase

In addition to the water phase described above, the antiperspirant compositions also include an oil phase. In some examples, the oil phase includes multiple structurants and one or multiple emulsifiers/surfactants/co-surfactants. In some examples, the oil phase also includes a skin emollient to improve the skin "feel" of the antiperspirant composition as it is applied to the skin.

The oil phase includes a microcrystalline wax:paraffin wax ratio of 1:1 to 1:5, which facilitates the prevention of the antiperspirant composition from solidifying and preventing the composition from leaving a white residue behind on the user's skin. Table 2 below illustrates a first example and a second example of an oil phase of a soft solid antiperspirant deodorant. Each of the ingredients and compound amounts is indicated in weight percents of the overall soft solid antiperspirant composition.

**(Table 2)**

| Function | Ingredients and Compounds | 1^{st} Example Wt% | 2^{nd} Example Wt% |
|---|---|---|---|
| Emollient | Finsolv TN - C12-15 Alkyl Benzoate | 8.868 | 10.094 |
| Structurant | Paraffin SP-674 | 6.611 | 7.665 |
| Structurant | Microcrystalline wax SP-18 | 5.389 | 4.335 |
| Carrier | PMX-246 - Cyclohexasiloxane | 5.247 | 3.644 |
| Emulsifier | Abil® EM 180 - Cetyl PEG/PPG-10/1 Dimethicone | 0.785 | 1.162 |
| Structurant | Performa V 343 Polymer - Synthetic Wax | 0.1 | 0.1 |
| Sum: | | 27 | 27 |

The Finsolv TN may be purchased from Innospec, which has an office in Littleton, Colorado, U.S.A. The paraffin and microcrystalline wax may be purchased from Strahl & Pitsch, which is headquarted in West Babylon, New York, U.S.A. The PMX-246 may be a Xiameter® product, which can be purchased from Dow Corning. The Abil® EM 180 may be purchased from Evonik, which is headquartered in Essen, Germany. The Performa V 343 Polymer can be purchased from New Phase Technologies, which is headquartered in Sugar Land, Texas, U.S.A.

Paraffin wax is a white or colorless soft solid that can be used as a lubricant. Paraffin wax has a mixture of hydrocarbon molecules containing between twenty and forty carbon atoms in unbranched alkanes chains. Paraffin wax SP-674 has a melting point of 68.9 °C to 72.8 °C.

The microcrystalline wax contains a high percentage of branched hydrocarbons chains and naphthenes. Further, microcrystalline wax has smaller crystals than paraffin wax. Also, the microcrystalline wax is generally more viscous and has a higher molecular weight and melting point than paraffin waxes. The branched hydrocarbons of microcrystalline waxes cause the microcrystalline waxes to exhibit higher elastic and adhesive characteristics than paraffin waxes. Generally, the microcrystalline waxes are more flexible than paraffin wax. Microcrystalline wax SP-18 has a melting point of 73.9 °C to 79.4 °C.

The microcrystalline wax:paraffin wax ratio may range from 1:1 to 1:5. In some examples, the ratio is approximately 1:2.5. Such a ratio and mixture of the microcrystalline wax and paraffin wax forms a non-continuous crystalline structure that prevents the antiperspirant composition from solidifying, which allows the antiperspirant composition to remain in a physical state well suited to be a cream or another type of soft solid. While the above examples from Table 2 have been described with reference to specific structurant, additional appropriate structurants may be added. For example, synthetic wax may be incorporated in the oil phase as an additional structurant.

An emulsifier is a substance that stabilizes an emulsion. The emulsifier incorporated into the oil phase can have a wide range of molecular weights. As such, emulsifiers are often described with reference to an average molecular weight, i.e., a mass average of all of the constituent molecules in the emulsifier. In the examples from Table 2, the emulsifier used is Abil® EM 180, which is a Cetyl PEG/PPG-10/1 Dimethicone product that has an average molecular weight of approximately 80,000 Daltons. The high molecular weight of the emulsifier may provide, at least in part, stability to the antiperspirant composition. For example, the Abil® EM 180's high molecular weight may hold the microcrystalline wax and the paraffin wax particles in place and help contribute to maintaining the structure of the antiperspirant composition.

Any appropriate emulsifier with an average molecular weight over 20,000 Daltons may be used in accordance with the principles described herein. In some exemplary compositions, the emulsifier's average molecular weight is over 60,000 Daltons. For example, the emulsifier may be silicone-based water-in-oil emulsifiers, poly-(C₂-C₃)alkylene glycol-modified silicones, dimethicone copolyol, bis-PEG-y dimethicone (with y=3-25, preferably 4-20), PEG/PPG-a/b dimethicone (wherein a and b mutually independently denote numbers from 2-30, for example 3-30, such as 14-18), bis-PEG/PPG-c/d dimethicone (wherein c and d mutually independently denote numbers from 10-25, for example 14-20, such as 14-16), and bis-PEG/PPG-e/f PEG/PPG-g/h dimethicone (wherein e, f, g and h mutually independently denote numbers from 10-20, for example 14-18, such as particularly preferably 16). Further silicone-based water-in-oil emulsifiers may be employed in accordance with the principles described herein are poly-(C₂-C₃)-alkylene glycol-modified silicones, which are hydrophobically modified with C₄-C₁₈ alkyl groups, for example cetyl PEG/PPG-10/1 dimethicone, alkyl methicone copolyols, and alkyl dimethicone ethoxy glucoside.

In some exemplary compositions, the antiperspirant composition further includes a supplemental emulsifier or co-surfactant. The supplemental emulsifier or co-surfactant is provided to enhance the stability of the emulsion and to reduce the stickiness of the product after application, so as to avoid phase separation prior to application on to the skin.

An emollient may be included in the oil phase. An emollient, also referred to as a moisturizer, is complex mixtures of chemical agents configured to make a user's skin softer and more pliable. The emollient accomplishes this by increasing the skin's water content through reduced evaporation. In the example of Table 2, the emollient is Finsolv TN. The emollient may have a 7.0 to 12.0 weight percent of the overall antiperspirant composition. However, any appropriate emollient and weight percent of the emollient may be used in accordance with the principles described herein.

In addition to the compounds identified above, the antiperspirant product may also include additives, such as those used in conventional antiperspirants. These additives include, but are not limited to, fragrances, including encapsulated fragrances, dyes, pigments, preservatives, antioxidants, moisturizers, and the like. These ingredients and/or compounds can be included in the antiperspirant composition in an amount of about 0.0 weight percent to about 20.0 weight percent. In an exemplary composition, a fragrance is provided in the antiperspirant composition in a weight percentage of less than about 2.0 weight percent, and is added to the combined phase before homogenization.

To prepare the oil phase, the components are combined and heated to form a single phase mixture. For example, the carrier and select ingredients and/or compounds of the oil phase may be first combined and heated, and the structurants and any other remaining ingredients and/or compounds may then be added when the other ingredients have been raised to a desired temperature. Such a procedure may more effectively melt the structurants, thereby saving time and energy consumption. The components may be heated to a maximum temperature of about 80.0 °C to about 82.0 °C. Further, the components of the oil phase can be mechanically agitated to promote integration, such as by moderate stirring. As such, the oil phase, when combined, includes about 25.0 weight percent to about 40.0 weight percent of the overall antiperspirant composition.

### Emulsion

In some exemplary compositions, the antiperspirant composition is prepared by combining the water phase with the oil phase. Any suitable form of mixing can be used to combine (mix and/or homogenize) the ingredients and compounds, such as high shear mixing, stirring, agitation, blending, or any combination thereof. The water phase may be slowly added to the oil phase at a temperature of about 78.0 °C while continuously stirring to promote thorough homogenization. Thereafter, fragrance may be added, followed by homogenization in a homogenizer.

The final mixture is poured into molds, and then allowed to cool to room temperature, for a time period such as 24 hours. As used herein, the term "allowed to cool" refers to exposing the mixture to room temperature for a time sufficient for the mixture to come to room temperature or exposing the mixture to a refrigerator or cooling room, fan, or other cooling mechanism that lowers the temperature of the mixture to room temperature. The methods described above for making the composition are well suited for making batch quantities of the composition. However, any appropriate sequence of addition and/or combination of the various components of the antiperspirant product may be used.

In some exemplary compositions, the ingredients and compounds of the water phase are combined at a first temperature to form a first mixture. Further, the ingredients of the oil phase are combined at a second temperature to form a second mixture. The first mixture and the second mixtures are combined using a mixing blade. The blended first and second mixtures are then homogenized to form the antiperspirant composition. In some exemplary compositions, homogenization reduces the particle sizes of the water phase and oil phase from approximately 30.0 to 40.0 microns to approximately 1.0 micron. The homogenized emulsion is then poured into a mold and allowed to cool in the mold at a third temperature that is lower than both the first and second temperatures.

Table 3 below illustrates a first example and a second example of an emulsion that uses a water phase that corresponds to the water phase of Table 1 and also uses an oil phase that corresponds to the oil phase of Table 2. Each of the ingredients and compounds amounts is indicated in weight percents of the overall soft solid antiperspirant composition.

**(Table 3)**

| Function | Ingredients | 1^{st} Example Wt% | 2^{nd} Example Wt% |
|---|---|---|---|
| | Water Phase | 71.1 | 71.1 |
| | Oil Phase | 27.0 | 27.0 |
| Fragrance | Fragrance | 1.9 | 1.9 |
| Sum: | | 100.0 | 100.0 |
| Hardness (gm force) | | 619.0 | 1117.7 |

The hardness is measured by a TA-XT2i Texture Analyzer, which can be purchased from Stable Mirco Systems, which is headquartered in Surrey, United Kingdon. The TA-XT2i Texture Analyzer is used to push a ½ inch ball probe (TA-18) through the antiperspirant composition for a distance of 15.0 millimeters at a rate of 2.7 millimeters/second. Tables 4 and 5 display the weight loss performance for the first and second examples of the emulsions that correspond with the emulsions in Table 3.

**(Table 4)**

| 1^{st} Example Weight loss % | 1 month | 2 month | 3 month |
|---|---|---|---|
| 4 °C | 0.12% | 0.15% | 0.15% |
| 25 °C | 0.23% | 0.48% | 0.58% |
| 40 °C | 0.50% | 0.68% | 1.32% |
| 45 °C | 0.48% | 1.20% | |

**(Table 5)**

| 2^{nd} Example Weight loss % | 1 month | 2 month | 3 month |
|---|---|---|---|
| 4 °C | 0.06% | 0.12% | 0.09% |
| 25 °C | 0.10% | 0.33% | 0.42% |
| 40 °C | 0.31% | 0.66% | 0.91% |
| 45 °C | 0.77% | 2.35% | |

Experimental results indicate that the combination of the microcrystalline wax/paraffin wax ratio and a higher molecular weight emulsifier contribute to reducing the white residue on skin as well as contribute to a dry feeling as a user applies the antiperspirant composition to the user's skin. Such results are shown in Table 6 below.

**(Table 6)**

| Function | Formula Summary | 3^{rd} Example | 4^{th} Example | 5^{th} Example | 6^{th} Example |
|---|---|---|---|---|---|
| | Ingredient/Compound | Wt% | Wt% | Wt% | Wt% |
| Active | AAZG-3109 | 67.11 | 67.11 | 67.11 | 67.11 |
| Carrier | Propylene Glycol | 3.99 | 3.99 | 3.99 | 3.99 |
| Carrier | PMX-245 | 9.70 | 9.70 | 9.70 | 9.70 |
| Emollient | Finsolv TN | 10.00 | 10.00 | 11.50 | 8.00 |
| Emulsifier | Abil® EM 90 | 0.80 | | | |
| Emulsifier | Abil® EM 97 | 0.40 | | | 0.60 |
| Emulsifier | Abil® EM 180 | | 1.20 | 1.20 | 0.60 |
| Structurant | Syncrowax | 6.00 | 6.00 | | |
| Structurant | Microcrystalline wax | | | 2.00 | 2.00 |
| Structurant | Paraffin | | | 6.00 | 6.00 |
| Structurant | Performa V 343 | 0.10 | 0.10 | 0.10 | 0.10 |
| Fragrance | Fragrance | 1.90 | 1.90 | 1.90 | 1.90 |
| | Sum: | 100.00 | 100.00 | 100.00 | 100.00 |
| | Application feel | Very wet | Not wet | Not wet | Slightly wet |
| | White residue on skin | no | Visible white | no | no |

Table 6 demonstrates that the fifth example does not have a wet application feel and does not leave behind a white residue on the user's skin. The fifth example has the appropriate microcrystalline wax/paraffin wax ratio as well as an emulsifier that has an average molecule weight of 20,000 Daltons.

In the 3^{rd} example, where Abil® EM 90 is used, which has an average molecular weight less than 20,000 Daltons, the resulting composition has the undesirable characteristic of feeling wet as the composition is applied to the user's skin. The fourth example uses the heavier weight emulsifier, but does not use the microcrystalline wax and the paraffin wax. The resulting composition of the fourth example is that the composition leaves behind a visible white residue on the user's skin. The sixth example uses the microcrystalline wax and the paraffin wax, but replaces a portion of the Abil® EM 180 with Abil® EM 97. The resulting composition of the sixth example was a composition that felt slightly wet to the user. Such experimental results illustrate that the unique combination of the microcrystalline wax structurant, the paraffin wax structurant, and the emulsifier with an average molecule weight of over 20,000 Daltons results in an antiperspirant composition in a soft solid molecular structure with a unique set of characteristics.

Further, while the principles described herein result in compositions that exhibit a dry application feel and do not leave behind white residue, the water in oil emulsion antiperspirant soft solids also result in slightly better sweat reduction than their solid stick counterparts. Thus, the principles described herein produce a quality antiperspirant product well suited for personal use.

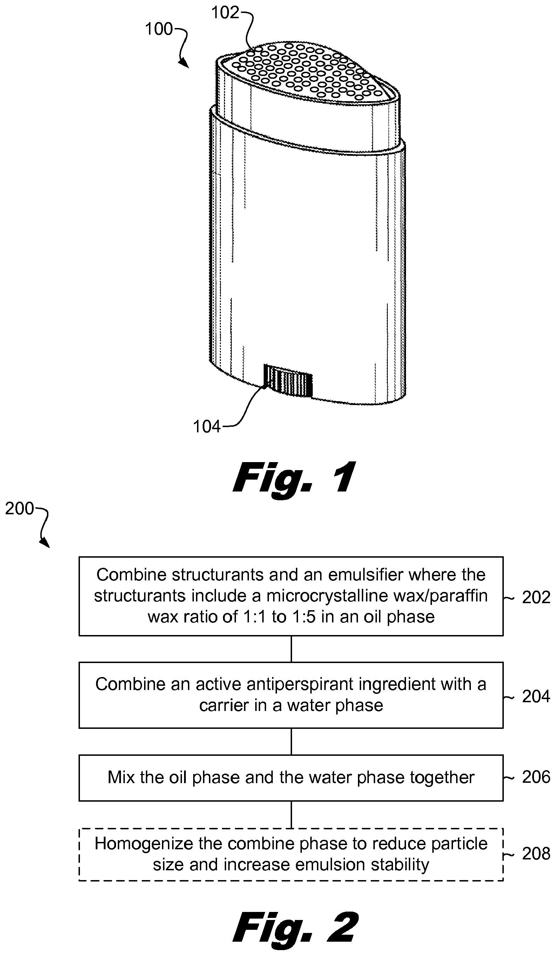

Now turning to FIG. 1, which is a diagram of a container (100) of an exemplary soft solid antiperspirant composition according to the principles described herein. In this example, the antiperspirant deodorant is a soft solid that is packaged in the container (100) that has a perforated opening (102). The soft solid can be pushed out of the perforated opening (102) by rotating a dial (104) positioned at the bottom of the container (100). As the dial (104) is rotated, a platform internal to the container (100) moves up and pushes the soft solid with it. Under such conditions, the soft solid is compressed between the perforated opening (102) and the internal platform, which causes the soft solid to behave as a fluid. Thus, the soft solid flows through the perforated opening such that it provides to a user the extruded soft solid for application to the user's appropriate target areas.

While the above examples have been described with reference to a specific type of container, any appropriate type of container may be used in accordance with the principles described herein. For example, the container may have soft sides that allow a user to squeeze the container to extrude some of the composition out of an opening of the container.

FIG. 2 is a diagram of an exemplary method (200) for making a soft solid antiperspirant composition according to the principles described herein. In this example, the method (200) includes combining (202) structurants and an emulsifier where the structurants include a microcrystalline wax/paraffin wax ratio of 1:1 to 1:5 in an oil phase, combining (204) an active antiperspirant ingredient and/or compound with a carrier in a water phase, and mixing (206) the oil phase and the water phase together. The method outlined in Fig. 2 also includes homogenizing (208) the combine phases to reduce particle size and to increase the emulsion's stability.

Combining the constituents of the oil phase and combining the constituents of the water phase may occur simultaneously. In other suitable methods, the water phase is combined first. In yet other examples, the oil phase is combined first.

The constituents of the water phase may be combined and heated to 65.0 °C. The combined water phase constituents may be moderately mixed with any appropriate mechanism. The constituents of the oil phase may be combined and heated to 80.0 °C. The combined oil phase constituents may be moderately mixed with any appropriate mechanism. In some suitable methods, the structurants may start to melt at 68.0 °C and be completely melted at 78.0 °C.

The water phase may be pumped into the oil phase with a Simon varistalic pump at a speed of 1 while the oil phase is continued to be mixed at 500 rotations per minute. The Simon varistalic pump can be purchased from Biotech Equipment Sales, Inc., which is located in San Francisco, California, U.S.A. After the water phase is completely added to the oil phase, the phases are continued to be mixed for at least 7.0 minutes at 77.0 °C to 79.0 °C at 500 rotations per minute. In some suitable methods, the phases are mixed for 10.0 minutes. A fragrance is added and mixed for 1.0 minute.

The composition is then homogenized for at least 50.0 seconds using a Silverson L4RT-A mixer at 4,000 rotations per minute. In some suitable methods, the composition is homogenized for 1.0 minutes at 5,000 rotations per minute. The Silverson L4RT-A mixer may be purchased from Silverson Machines, Inc. headquartered in Chesham, United Kingdom. Homogenizing reduces the mixed phase particles into smaller sizes. One characteristic that may result from homogenizing the composition is increased stability. Another characteristic may include that the phase particles are reduced to about one micron through homogenizing. Next, the composition is poured into a canister and allowed to cool down at room temperature.

Accordingly, various exemplary antiperspirant compositions exhibiting desirable look and feel characteristics have been disclosed. The antiperspirant compositions are desirably processed and manufactured with emulsifiers of heavier average molecular weight and use unique structurants. Various embodiments of methods for manufacturing the same have also been provided.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way.

## Claims

1. An antiperspirant composition, comprising:
a water-in-oil emulsion comprising structurants and an emulsifier, wherein the structurants comprise microcrystalline wax and paraffin wax in a microcrystalline wax:paraffin wax ratio of 1 : 1 to 1 :5; wherein the paraffin wax is 6.0 to 8.0 weight percent of the composition; wherein the microcrystalline wax is 4.0 to 6.0 weight percent of the composition.

2. The composition of claim 1, wherein the microcrystalline wax:paraffin wax ratio is 1:2.5.

3. The composition of claim 1, wherein the emulsifier includes dimethicone copolyol.

4. The composition of claim 3, wherein the dimethicone copolyol has an average molecular weight of over 20,000 Daltons.

5. The composition of claim 1, wherein the emulsifier is 0.5 to 1.5 weight percent of the composition.

6. The composition of claim 1, wherein the structurants and the emulsifier are each part of an oil phase that is mixed with a water phase, wherein the water phase is 63.0 to 75.0 weight percent of the composition.

7. The composition of claim 1, wherein the composition is a soft solid.

8. The composition of claim 1, further comprising an active antiperspirant that constitutes between 18.0 and 25.0 weight percent of the composition.

## Patentansprüche

1. Antitranspirtant-Zusammensetzung, die Folgendes umfasst:
eine Wasser-in-ÖI-Emulsion, die Strukturmittel und einen Emulgator umfasst, wobei die Strukturmittel mikrokristallines Wachs und Paraffinwachs in einem Verhältnis von mikrokristallinem Wachs zu Paraffinwachs von 1:1 bis 1:5 umfassen;
wobei das Paraffinwachs 6,0 bis 8,0 Gew.-% der Zusammensetzung beträgt;
wobei das mikrokristalline Wachs 4,0 bis 6,0 Gew.-% der Zusammensetzung beträgt.

2. Zusammensetzung nach Anspruch 1, wobei das Verhältnis von mikrokristallinem Wachs zu Paraffinwachs 1:2,5 beträgt.

3. Zusammensetzung nach Anspruch 1, wobei der Emulgator Dimethiconcopolyol einschließt.

4. Zusammensetzung nach Anspruch 3, wobei das Dimethicon-Copolyol ein durchschnittliches Molekulargewicht von über 20.000 Dalton aufweist.

5. Zusammensetzung nach Anspruch 1, wobei der Emulgator 0,5 bis 1,5 Gew.-% der Zusammensetzung beträgt.

6. Zusammensetzung nach Anspruch 1, wobei die Strukturmittel und der Emulgator jeweils Teil einer Ölphase sind, die mit einer Wasserphase vermischt ist, wobei die Wasserphase 63,0 bis 75,0 Gew.-% der Zusammensetzung beträgt.

7. Zusammensetzung nach Anspruch 1, wobei die Zusammensetzung ein weicher Feststoff ist.

8. Zusammensetzung nach Anspruch 1, ferner umfassend ein aktives Antitranspirant, das zwischen 18,0 und 25,0 Gew.-% der Zusammensetzung ausmacht.

## Revendications

1. Composition anti-transpirante comprenant :
une émulsion eau-dans-huile comprenant des structurants et un émulsifiant, les structurants comprenant une cire microcristalline et une cire de paraffine dans un rapport cire microcristalline:cire de paraffine de 1:1 à 1:5 ;
la cire de paraffine représentant 6,0 à 8,0 % en poids de la composition ;
la cire microcristalline représentant 4,0 à 6,0 % en poids de la composition.

2. Composition selon la revendication 1, dans laquelle le rapport cire microcristalline:cire de paraffine est de 1:2,5.

3. Composition selon la revendication 1, dans laquelle l'émulsifiant comprend du copolyol de diméthicone.

4. Composition selon la revendication 3, dans laquelle le copolyol de diméthicone a un poids moléculaire moyen supérieur à 20 000 daltons.

5. Composition selon la revendication 1, dans laquelle l'émulsifiant représente 0,5 à 1,5 % en poids de la composition.

6. Composition selon la revendication 1, dans laquelle les structurants et l'émulsifiant font chacun partie d'une phase huileuse qui est mélangée à une phase aqueuse, la phase aqueuse représentant 63,0 à 75,0 % en poids de la composition.

7. Composition selon la revendication 1, dans laquelle la composition est une matière solide molle.

8. Composition selon la revendication 1, comprenant en outre un anti-transpirant actif qui représente entre 18,0 et 25,0 % en poids de la composition.
